# EUROPEAN PATENT SPECIFICATION

(11) **EP 0 403 159 B1**
(45) Date of publication and mention of the grant of the patent: **01.03.2000**
(21) Application number: 90306204.0
(22) Date of filing: 07.06.1990
(51) Int. Cl.: C07D 233/58, C07D 233/70, C07D 233/82, C07D 401/12, C07D 403/12, C07D 405/12, C07D 409/12, A61K 31/415

(54) **Imidazolyl-alkenoic acids**
Imidazoalkensäure
Acides imidazoalcénoiques

(30) Priority: 14.06.1989 US 366079; 06.04.1990 US 506412
(43) Date of publication of application: 19.12.1990
(60) Divisional application: 99115614.2
(73) Proprietor: SMITHKLINE BEECHAM CORPORATION, Philadelphia Pennsylvania 19101 (US)
(72) Inventor: Finkelstein, Joseph Alan, Philadelphia, PA 19151 (US); Keenan, Richard McCulloch, Malvern, PA 19355 (US); Weinstock, Joseph, Phoenixville, PA 19460 (US)
(74) Representative: Waters, David Martin, Dr.

(56) References cited:
- EP-A- 0 028 834
- EP-A- 0 253 310
- EP-A- 0 294 973
- EP-A- 0 302 603
- EP-A- 0 324 377

## Description

The present invention relates to new imidazolyl-alkenoic acids which are angiotensin II receptor antagonists and are useful in regulating hypertension induced or exacerbated by angiotensin II, and in the treatment of congestive heart failure, renal failure, and glaucoma. This invention also relates to pharmaceutical compositions containing these compounds and methods for using these compounds as antagonists of angiotensin II, as antihypertensive agents and as agents for treating congestive heart failure, renal failure, and glaucoma.

### BACKGROUND OF THE INVENTION

The class of peptide pressor hormone known as angiotensin is responsible for a vasopressor action that is implicated in the etiology of hypertension in man. Inappropriate activity of the renin-angiotensin systems appears to be a key element in essential hypertension, congestive heart failure and in some forms of renal disease. In addition to a direct action on arteries and arterioles, angiotensin II (AII), being one of the most potent endogenous vasoconstrictors known, exerts stimulation on the release of aldosterone from the adrenal cortex. Therefore, the renin-angiotensin system, by virtue of its participation in the control of renal sodium handling, plays an important role in cardiovascular hemeostasis.

Interruption of the renin-angiotensin system with converting enzyme inhibitors, such as captopril, has proved to be clinically useful in the treatment of hypertension and congestive heart failure (Abrams, W.B., et al., (1984), Federation Proc., 43, 1314). The most direct approach towards inhibition of the renin-angiotensin system would block the action of AII at the receptor. Compelling evidence suggests that AII also contributes to renal vasoconstriction and sodium retention that is characteristic of a number of disorders such as heart failure, cirrhosis and complications of pregnancy (Hollenberg, N.K., (1984), J. Cardiovas. Pharmacol., 6, S176). In addition, recent animal studies suggest that inhibition of the renin-angiotensin system may be beneficial in halting or slowing the progression of chronic renal failure (Anderson, S., et al., (1985), J. Clin. Invest., 76, 612). Also, a recent patent application (South African Patent Application No. 87/01,653) claims that AII antagonists are useful as agents for reducing and controlling elevated intraocular pressure, especially glaucoma, in mammals.

The compounds of this invention inhibit, block and antagonize the action of the hormone AII, and are therefore useful in regulating and moderating angiotensin induced hypertension, congestive heart failure, renal failure and other disorders attributed to the actions of AII. When compounds of this invention are administered to mammals, the elevated blood pressure due to AII is reduced and other manifestations based on AII intercession are minimized and controlled. Compounds of this invention are also expected to exhibit diuretic activity.

Recognition of the importance of blocking and inhibiting the actions of AII has stimulated other efforts to synthesize antagonists of AII. The following references have disclosed imidazole derivatives which are described as having AII blocking activity and useful as hypotensive agents.

Furukawa et al., U.S. Patent 4,340,598 discloses imidazol-5-yl acetic acids and imidazol-5-yl-propanoic acids. Specifically, the discloser includes 1-benzyl-2-n-butyl-5-chloroimidazole-4-acetic acid and 1-benzyl-2-phenyl-5-chloro-imidazole-4-propanoic acid.

Furukawa, et al., U.S. Patent 4,355,040 discloses substituted imidazole-5-acetic acid derivatives. A compound specifically disclosed is 1-(2-chlorobenzyl)-2-n-butyl-4-chloroimidazole-5-acetic acid.

Carini et al. in EP 253,310 disclose certain imidazolylpropenoic acids. Two intermediates described in this patent are ethyl 3-[1-(4-nitrobenzyl)-2-butyl-4-chloroimidazol-5-yl]propenoate and ethyl 3-[2-butyl-4-chloro-1-(4-aminobenzyl)imidazol-5-yl]propenoate.

Also, Wareing, in PCT/EP 86/00297, discloses as intermediates certain imidazolylpropenoate compounds. On page 62, Formula (CX) is ethyl 3-[1-(4-fluorophenyl)-4-isopropyl-2-phenyl-1H-imidazol-5-yl]-2-propenoate.

### DESCRIPTION OF THE INVENTION

The present invention relates to the blocker of angiotensin II receptors 3-[2-n-butyl-1-{(4-carboxyphenyl)methyl}-1H-imidazol-5-yl]-2-(2-thienyl)methyl-2-propenoic acid, represented by the following Formula (I): 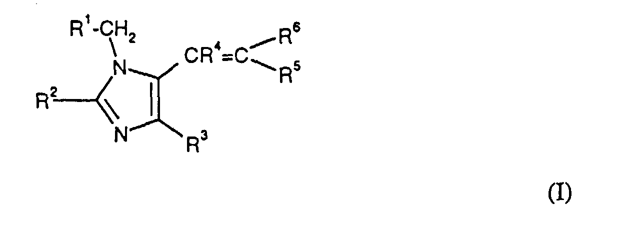 in which:
R¹ is 4-carboxyphenyl;
R² is n-butyl;
X is a single bond;
R³ is hydrogen;
R⁴ is hydrogen;
R⁵ is 2-thienyl-CH₂-; and
R⁶ is -COOH;
   or a pharmaceutically acceptable salt thereof.

The E isomer (trans stereochemistry of the carboxy and imidazole groups) is generally more active and thus, is preferred over the Z isomer (cis).

As used herein, the terms alkyl and alkoxy mean carbon chains which are branched or unbranched with the length of the chain determined by the descriptor preceding it.

A particular compound of the invention includes (E)-3-[2-n-butyl-1-{(4-carboxyphenyl)methyl}-1H-imidazol-5-yl]-2-(2-thienyl)methyl-2-propenoic acid, or a pharmaceutically acceptable salt thereof.

The invention also relates to pharmaceutical compositions comprising a pharmaceutical carrier and an effective amount of a compound of Formula (I).

Also included in the present invention are methods for antagonizing angiotensin II receptors which comprises administering to a subject in need thereof an effective amount of a compound of Formula (I). Methods of producing antihypertensive activity and methods of treating congestive heart failure, glaucoma, and renal failure by administering these compounds are also included in this invention.

The compounds of this invention are prepared by procedures described herein and illustrated by the examples. Reagents, protecting groups and functionality on the imidazole and other fragments of the molecule must be consistent with the proposed chemical transformations. Steps in the synthesis must be compatible with the functional groups and the protecting groups on the imidazole and other parts of the molecule.

The starting material, 2-R²-imidazole, is known to the art (J. Org. Chem. 45:4038, 1980) or is synthesized by known procedures. For example, imidazole is converted to 2-n-butylimidazole by reacting imidazole with triethylorthoformate and p-toluenesulfonic acid to give 1-diethoxyorthoamide imidazole and then treating with n-butyl lithium to give the 2-lithium derivative of the orthoamide and alkylating with n-butyl iodide in a suitable solvent, such as tetrahydrofuran (THF).

The following procedure is useful for the preparation of the compounds of Formula (I).

The 1-R¹CH₂-group is incorporated onto the 2-R²-imidazole by known procedures, for example, by reaction with an R¹-CH₂ halide, mesylate or acetate, such as 2-chlorobenzyl bromide, in a suitable solvent, such as dimethylformamide (DMF), in the presence of a suitable acid acceptor, such as sodium alkylate, potassium or sodium carbonate, or a metal hydride, preferably sodium hydride at a reaction temperature of 25°C to 100°C, preferably 50°C. The resulting 1-R¹CH₂-2-R²-imidazole is hydroxymethylated in the 5-position, for example, by reacting with formaldehyde in the presence of sodium acetate in acetic acid to provide the 1-R¹CH₂-2-R²-5-hydroxymethylimidazole intermediates.

Alternatively, the 1-R¹CH₂-2-R²-5-hydroxymethylimidazole intermediates are prepared by reacting an imido ether, R²-C(=NH)-O-alkyl, such as valeramidine methyl ether, with dihydroxyacetone in liquid ammonia under pressure to give 2-R²-5-hydroxymethylimidazole. This intermediate is reacted with acetic anhydride to give 1-acetyl-5-acetoxymethyl-2-R²-imidazole. The diacetate intermediate is N-alkylated, for example, using 2-chlorobenzyl triflate and the resulting 1-R¹CH₂-2-R²-5-acetoxymethylimidazole is treated with aqueous base, such as 10% sodium hydroxide solution, to give the 1-R¹CH₂-2-R²-5-hydroxymethylimidazole intermediate.

The hydroxymethyl group of the hereinbefore prepared intermediate is oxidized to an aldehyde by treatment with a suitable reagent, such as anhydrous chromic acid-silica gel in tetrahydrofuran or, preferably, with activated manganese dioxide, in a suitable solvent, such as benzene or toluene, or preferably methylene chloride, at a temperature of 25°C to 140°C, preferably at 25°C. The 1-R¹CH₂-2-R²-imidazol-5-carboxaldehyde is reacted with an appropriate phosphonate. The phosphonates are prepared, for example, from trialkyl phosphonoacetates by alkylation with an appropriate halide, mesylate or acetate in the presence of a suitable base, such as sodium hydride, in a suitable solvent, preferably glyme at a reaction temperature of 25°C to 110°C, preferably at 55°C. The reaction of the imidazol-5-carboxaldehydes with the phosphonates is performed in the presence of a suitable base, such as a metal alkoxide, lithium hydride or preferably sodium hydride, in a suitable solvent, such as ethanol, methanol, ether, dioxane, tetrahydrofuran, or preferably glyme, at a reaction temperature of 10°C to 50°C, preferably at 25°C, to provide a variable mixture of trans and cis, e.g., (E) and (Z), 1-R¹CH₂-2-R²-5-CH=C(R⁵)-(COO-alkyl)-imidazoles. These isomers are readily separated by chromatography over silica gel in suitable solvent systems, preferably hexane in ethyl acetate mixtures. The esters are hydrolyzed to the acids, 1-R¹-CH₂-2-R²-5-CH=C(R⁵)COOH-imidazoles, using bases, such as potassium hydroxide, lithium hydroxide or sodium hydroxide, in a suitable solvent system, such as, for example, aqueous alcohols or diglyme. The trans and cis structures of the acids are readily determined by NMR by the NOE protocol, as well as by the biological activities since, generally, the trans (E) isomeric acids are the more potent isomers.

Alternatively, the 1-R¹CH₂-2-R²-imidazol-5-carboxaldehydes are prepared by the following procedure. Starting 2-R²-imidazol-4-carboxaldehydes are reacted with an N-alkylating protecting reagent, such as chloromethyl pivalate (POM-Cl), in the presence of a base, such as dimethylformamide, at a temperature of 20°C to 50°C, preferably at 25°C, to give N-alkylation (e.g. POM-derivation) on the least hindered nitrogen atom of the imidazole nucleus. The 1-R¹CH₂-group is incorporated onto the imidazole by N-alkylation of the above prepared aldehyde with a halomethylbenzene compounds, such as methyl 4-bromomethyl-3-chlorobenzoate, at a temperature of 80°C to 125°C, preferably at 100°C. The protecting group on the 3-nitrogen of the imidazole ring is removed by base hydrolysis, for example using a biphasic mixture of ethyl acetate and aqueous sodium carbonate, to give 1-R¹CH₂-2-R²-imidazole-5-carboxaldehyde compounds. The Formula (I) compounds can be prepared from these 5-carboxaldehyde compounds by the methods described above.

Compounds of Formula (I) are also prepared by the following procedure. The 2-R²-imidazole starting materials are reacted with trimethylsilylethoxymethyl (SEM) chloride to give 1-(trimethylsilyl) ethoxymethyl-2-R²-imidazole. The reaction is carried out, for example, in the presence of sodium hydride in a solvent such as dimethylformamide, The 5-tributyltin derivatives are prepared by lithiation with, for example, butyllithium in a suitable solvent, preferably diethyl ether, followed by treatment of the lithio imidazole derivative with a tributyltin halide, preferably tri-N-butyltin chloride, at -10°C to 35°C, preferably at 25°C. The 1-SEM-2-R²-5-tributyltinimidazole is coupled with an α,β-unsaturated acid ester having a leaving group on the β-position, such as a halide or trifluoromethanesulfonyloxy group, for example, BrCR⁴=C(R⁵)(COOalkyl), in the presence of a phosphine ligand, such as bis(diphenylphosphino)propane, or triphenylphosphine and a palladium (II) compound, or preferably tetrakis(triphenylphosphine)-palladium(0), with or without a base, such as tributylamine, at a temperature of 50°C to 150°C, preferably at 120°C. Both the (E) and (Z) olefinic isomers are prepared by this procedure, and the isomeric esters are readily separated by chromatography over silica gel. The 1-SEM group from the (E) and (Z) isomers is hydrolyzed with acid, for example, aqueous hydrochloric, in a suitable alcoholic solvent, such as methanol or ethanol, and the 1-unsubstituted imidazole derivatives are converted to the 1-t-butoxycarbonyl (t-BOC) imidazoles with di-t-butyl dicarbonate (Hoppe-Seyler's Z. Physiol. Chem., (1976), 357, 1651). The t-BOC esters are alkylated and hydrolyzed with, for example, 2-chlorobenzyl-O-triflate in the presence of a suitable base, preferably diisopropylethylamine, in a suitable solvent, preferably methylene chloride, to afford the 1-(2-chlorophenyl)methylimidazole derivatives (esters). The (E) and (Z) isomers are hydrolyzed to the (E) and (Z) acids by the method described above.

Compounds of Formula (I) are also prepared by the following procedure. The 1-R¹CH₂-2-R²-imidazole-5-carboxaldehydes, prepared as described above, are reacted with a substituted half-acid, half-ester derivative of a malonate, such as ethyl 2-carboxy-3-(2-thienyl)propionate, in the presence of a base, such as piperidine, in a suitable solvent, such as toluene, at a temperature of 80°C to 110°C, preferably at 100°C. The resulting 1-R¹CH₂-2-R²-5-CH=C(R⁵)COO alkylimidazoles are hydrolyzed to the corresponding Formula (I) acid compounds by alkaline hydrolysis as described above.

Compounds of Formula (I) are also prepared as follows. The 1-R¹-CH₂-2-R²-imidazol-5-carboxaldehydes are treated with lithium derivative of a substituted ethyl or methyl ester. These lithio derivatives are prepared from the reaction of lithium diisopropylamide in a suitable solvent, preferably tetrahydrofuran, with an acid ester, such as ROOC-CH₂-CH₂-(2-thienyl), to generate the α-lithio derivatives at -78°C to -10°C, preferably at -78°C, which are then treated with the imidazol-carboxaldehyde. The intermediate β-hydroxy group of the imidazole ester is converted to a mesylate or an acetate and the mesylate, or preferably the acetate, is heated in a suitable solvent, such as toluene, with one to two equivalents of 1,8-diazobicyclo[5.4.0]undec-7-ene, at 50 to 110°C, preferably at 80°C, to afford ester compounds of Formula (I) such as 3-(imidazol-5-yl)-2-(2-thienyl)methyl-2-propenoic acid esters. The (E) isomer is the predominate olefinic isomer. The acids are prepared from the esters by the method described above.

Compounds of Formula (I) are formed from the corresponding compounds in which the R¹group is 4-(CO₂C₁₋₄alkyl)phenyl using basic hydrolysis, such as aqueous sodium or potassium hydroxide in methanol or ethanol, or using acidic hydrolysis, such as aqueous hydrochloric acid.

Pharmaceutically acceptable acid addition salts of compounds of Formula (I) are formed with appropriate organic or inorganic acids by methods known in the art. For example, the base is reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Representative examples of suitable acids are maleic, fumaric, benzoic, ascorbic, pamoic, succinic, bismethylene-salicyclic, methanesulfonic, ethanedisulfonic, acetic, propionic, tartaric, salicyclic, citric, gluconic, aspartic, stearic, palmitic, itaconic, glycolic, p-aminobenzoic, glutamic, benzenesulfonic, hydrochloric, hydrobromic, sulfuric, cyclohexyl-sulfamic, phosphoric and nitric acids.

Pharmaceutically acceptable base addition salts of compounds of Formula (I) are prepared by known methods from organic and inorganic bases, including nontoxic alkali metal and alkaline earth bases, for example, calcium, lithium, sodium, and potassium hydroxide; ammonium hydroxide, and nontoxic organic bases, such as triethylamine, butylamine, piperazine, and (trihydroxymethyl)methylamine.

Angiotensin II antagonist activity of the compounds of Formula (I) is assessed by in vitro and in vivo methods. In vitro antagonist activity is determined by the ability of the compounds to compete with ¹²⁵I-angiotensin II for binding to vascular angiotensin II receptors and by their ability to antagonize the contractile response to angiotensin II in the isolated rabbit aorta. In vivo activity is evaluated by the efficacy of the compounds to inhibit the pressor response to exogenous angiotensin II in conscious rats and to lower blood pressure in a rat model of renin dependent hypertension.

### Binding

The radioligand binding assay is a modification of a method previously described in detail (Gunther et al., Circ. Res. 47:278, 1980). A particular fraction from rat mesenteric arteries is incubated in Tris buffer with 80 pM of ¹²⁵I-angiotensin II with or without angiotensin II antagonists for 1 hour at 25°C. The incubation is terminated by rapid filtration and receptor bound ¹²⁵I-angiotensin II trapped on the filter is quantitated with a gamma counter. The potency of angiotensin II antagonists is expressed as the IC₅₀ which is the concentration of antagonist needed to displace 50% of the total specifically bound angiotensin II. Exemplary of the IC₅₀ of compounds of the invention (E isomers) is about 0.1 nM to about 100 µM.

### Aorta

The ability of the compounds to antagonize angiotensin II induced vasoconstriction is examined in the rabbit aorta. Ring segments are cut from the rabbit thoracic aorta and suspended in organ baths containing physiological salt solution. The ring segments are mounted over metal supports and attached to force displacement transducers which are connected to a recorder. Cumulative concentration response curves to angiotensin II are performed in the absence of antagonist or following a 30-minute incubation with antagonist Antagonist disassociation constants (K_{B}) are calculated by the dose ratio method using the mean effective concentrations. Exemplary of the K_{B} of compounds of the invention (E isomers) is about 0.1 nM to about 30 µM.

### Inhibition of pressor response to angiotensin II in conscious rats

Rats are prepared with indwelling femoral arterial and venous catheters and a stomach tube (Gellai et al., Kidney Int. 15:419, 1979). Two to three days following surgery the rats are placed in a restrainer and blood pressure is continuously monitored from the arterial catheter with a pressure transducer and recorded on a polygraph. The change in mean arterial pressure in response to intravenous injections of 250 mg/kg angiotensin II is compared at various time points prior to and following the administration of the compounds intravenously or orally at doses of 0.1 to 300 mg/kg. The dose of compound needed to produce 50% inhibition of the control response to angiotensin II (IC₅₀) is used to estimate the potency of the compounds. The IC₅₀ of (E)-3-[2-n-butyl-01-{(2-chlorophenyl)methyl}-1H-imidazol-5-yl]-2-(2-thienyl)methyl-2-propenoic acid is 3.60 mg/kg i.v. and 44.00 mg/kg orally.

### Antihypertensive activity

The antihypertensive activity of the compounds is measured by their ability to reduce mean arterial pressure in conscious rats made renin-dependent hypertensive by ligation of the left renal artery (Cangiano et al., J. Pharmacol. Exp. Ther. 208:310, 1979). Renal artery ligated rats are prepared with indwelling catheters as described above. Seven to eight days following renal artery ligation, the time at which plasma renin levels are highest, the conscious rats are placed in restrainers and mean arterial pressure is continuously recorded prior to and following the administration of the compounds intravenously or orally. The dose of compound needed to reduce mean arterial pressure by 30 mm Hg (IC₅₀) is used as an estimate of potency. The IC₅₀ of (E)-3-[2-n-butyl-1-{2-chlorophenyl)methyl}-1H-imidazol-5-yl]-2-(2-thienyl)methyl-20-propenoic acid is 1.80 mg/.kg i.v. and 8.0 mg/kg orally.

The intraocular pressure lowering effects employed in this invention may be measured by the procedure described by Watkins, et al., J. Ocular Pharmacol., 1 (2):161-168 (1985).

The compounds of Formula (I) are incorporated into convenient dosage forms, such as injectable preparations, or for orally active compounds, capsules or tablets. Solid or liquid pharmaceutical carriers are employed. Solid carriers include starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid, such as an ampoule, or an aqueous or nonaqueous liquid suspension.

For topical ophthalmologic administration, the pharmaceutical compositions adapted include solutions, suspensions, ointments, and solid inserts. Typical pharmaceutically acceptable carriers are, for example, water, mixtures of water and water-miscible solvents such as lower alkanols or vegetable oils, and water soluble ophthalmologically acceptable non-toxic polymers, for example, cellulose derivatives such as methyl cellulose. The pharmaceutical preparation may also contain non-toxic auxiliary substances such as emulsifying, preserving wetting, and bodying agents, as for example, polyethylene glycols; antibacterial components, such as quaternary ammonium compounds; buffering ingredients, such as alkali metal chloride; antioxidants, such as sodium metabisulfite; and other conventional ingredients, such as sorbitan monolaurate.

Additionally, suitable ophthalmic vehicles may be used as carrier media for the present purpose including conventional phosphate buffer vehicle systems.

The pharmaceutical preparation may also be in the form of a solid insert. For example, one may use a solid water soluble polymer as the carrier for the medicament. Solid water insoluble inserts, such as those prepared from ethylene vinyl acetate copolymer, may also be utilized.

The pharmaceutical preparations are made following conventional techniques of a pharmaceutical chemist involving mixing, granulating, and compressing, when necessary, for tablet forms, or mixing, filling and dissolving the ingredients, as appropriate, to give the desired oral, parenteral, or topical products.

Doses of the compounds of Formula (I) in a pharmaceutical dosage unit as described above will be an efficacious, nontoxic quantity selected from the range of .01 - 200 mg/kg of active compound, preferably 1 - 100 mg/kg. The selected dose is administered to a human patient in need of angiotensin II receptor antagonism from 1-6 times daily, orally, rectally, topically, by injection, or continuously by infusion. Oral dosage units for human administration preferably contain from 1 to 500 mg of active compound. Preferably, lower dosages are used for parenteral administration. Oral administration, at higher dosages, however, also can be used when safe and the active compound in an amount selected from 0.0001 to 0.1 (w/v %), preferably from 0.0001 to 0.01. As a topical dosage unit form, an amount of active compound from between 50 ng to 0.05 mg, preferably 50 ng to 5 µg, is applied to the human eye.

The method of this invention of antagonizing angiotensin II receptors in mammals, including humans, comprises administering to a subject in need of such antagonism an effective amount of a compound of Formula (I). The method of this invention of producing antihypertensive activity and the method of treating congestive heart failure, glaucoma, and renal failure comprise administering a compound of Formula (I) to a subject in need thereof an effective amount to produce said activity.

Contemplated equivalents of Formula (I) compounds are compounds otherwise corresponding thereto wherein substituents have been added to any of the unsubstituted positions of the Formula (I) compounds provided such compounds have the pharmaceutical utility of Formula (I) compounds.

The following examples illustrate preparation of compounds and pharmaceutical compositions of this invention. The examples are not intended to limit the scope of this invention as described hereinabove and as claimed below.

### Example 1

### (E)-3-[2-n-Butyl-1-{(2-chlorophenyl)methyl}-1H-imidazol-5-yl]-2-(2-thienyl)methyl-2-propenoic acid

### (ii) 2-n-butyl-1-(2-chlorophenyl)methyl-5-hydroxymethyl-1H-imidazole

### Method 2

A mixture of valeramidine methyl ether hydrochloride (250 g, 1.66 mol) and dihydroxyacetone (150 g, 0.83 mol) dissolved in liquid ammonia was allowed to stand overnight at room temperature in a pressure vessel, and then heated at 65°C for 4 hours at 375 psi. The ammonia was allowed to evaporate, and the residue was dissolved in methanol (3L). The resulting slurry was refluxed with added acetonitrile (1L). The solution was decanted from the solid ammonium chloride while hot. This procedure was repeated, and the combined acetonitrile extracts were treated with charcoal, filtered hot and the filtrate was concentrated in vacuum to give the dark oil, 2-n-butyl-5-hydroxymethylimidazole (253 g, 1.63 mol, 98%).

This crude alcohol (253 g) was treated with acetic anhydride (400 mL) at -15°C and then was allowed to warm to ambient temperature with stirring, and then stirred an additional 19 hours. The acetic anhydride was evaporated at reduced pressure, the residue taken up in methylene chloride, and the organic phase was washed with 5% sodium bicarbonate solution and water. The extract was dried over sodium sulfate and concentrated to give 323 g (83%) of 1-acetyl-4-acetoxymethyl-2-n-butylimidazole.

This diacetate was N-alkylated by the following procedure. To a solution of triflic anhydride (120 mL, 0.71 mol) in methylene chloride (200 mL) at -78°C under argon was added a solution of diisopropyl ethylamine (128 mL, 0.73 mol) and 2-chlorobenzyl alcohol (104 g, 0.72 mol) in methylene chloride (350 mL) over a period of 20 minutes. After being stirred an additional 20 minutes at -78°C, this solution was then treated with 1-acetyl-4-acetomethyl-2-n-butylimidazole (146 g, 0.61 mol) dissolved in methylene chloride (300 mL) over a 20-minute interval. The mixture was then stirred at ambient temperature for 18 hours and the solvents were evaporated. The residual 2-n-butyl-5-acetomethyl-1-(2-chlorophenyl)methyl-1H-imidazole (250 g) in methanol (200 mL) was treated with 10% sodium hydroxide solution (700 mL) and the mixture was heated on a steam bath for 4 hours. After cooling, methylene chloride was added, the organic phase was separated, washed with water, dried and concentrated. The residue was dissolved in ether, cooled, and seeded to give the crude product. Recrystallization from ethyl acetate gave 176 g of 2-n-butyl-1-(2-chlorophenyl)methyl-5-hydroxymethyl-1H-imidazole; mp 138-140°C.

### (iii) 2-n-butyl-1-(2-chlorophenyl)methyl-1H-imidazol-5-carboxaldehyde

A solution of 2-n-butyl-1-(2-chlorophenyl)methyl-5-hydroxymethyl-1H-imidazole (5.4 g, 0.0194 mol) in toluene (25 mL) was added to a suspension of activated manganese dioxide (27 g) in methylene chloride (325 mL). The suspension was stirred at room temperature for 17 hours. The solids were filtered and the filtrate concentrated and flash chromatographed over silica gel with 6:4 hexane/ethyl acetate to afford 4.16 g (78%) of 2-n-butyl-1-(2-chlorophenyl)methyl-1H-imidazol-5-carboxaldehyde, as an oil. NMR and IR were consistent with the structure.

### (iv) (E)-[2-n-butyl-1-{(2-chlorophenyl)methyl}-1H-imidazol-5-yl]-2-(2-thienyl)methyl-2-propenoic acid

### Method A

### (a) trimethyl 3-(2-thienyl)-2-phosphonopropionate

To a solution of 2-thiophenemethanol (2.28 g, 0.02 mol) in carbon tetrachloride (25 mL) was added triphenylphosphine (6.81 g, 0.026 mol), and the solution was refluxed for 3 hours. The cooled reaction mixture was diluted with hexane (60 mL), chilled and filtered. The concentrated filtrate (4.6 g) was flash chromatographed over silica gel with 7:3 hexane/ethyl acetate to provide 2-chloromethylthiophene (1.52 g, 57%) as an oil.

A suspension of sodium hydride (0.271 g, 11.3 mmol) in dry glyme (40 mL) under argon was treated dropwise with trimethyl phosphonoacetate (1.87 g, 10.3 mmol) in glyme (5 mL). The resulting mixture was stirred at room temperature for 1.5 hours. Then 2-chloromethylthiophene (1.5 g, 11.3 mmol) was added, and the mixture was stirred at 65°C for 18 hours. The reaction was partitioned between water and ethyl acetate, and the organic layer was washed with water and brine, dried with anhydrous magnesium sulfate and concentrated to 1.9 g of an oil. This was chromatographed over silica gel 4:1 ethylacetate/hexane to afford 800 mg (28%) of trimethyl 3-(2-thienyl)-2-phosphonopropionate.

### (b) methyl (E)-3-[2-n-butyl-1-{(2-chlorophenyl)methyl}-1H-imidazol-5-yl]-2-(2-thienyl)methyl-2-propenoate

To a suspension of sodium hydride (69 mg, 2.87 mmol) in glyme (5 mL) was added dropwise a solution of trimethyl 3-(2-thienyl)-2-phosphonopropionate in glyme (3 mL) under an atmosphere of argon. When the gas evolution had subsided, the mixture was heated to 50°C for 15 minutes. A solution of 2-n-butyl-1-(2-chlorophenyl)methyl-1H-imidazol-5-carboxaldehyde (0.53 g, 1.92 mmol) in glyme (3 mL) was added, and the mixture was stirred at 60-65°C for 5 hours. The cooled reaction was partitioned between water and ethyl acetate, and the organic layer was washed with water, dried, concentrated and flash chromatographed over silica gel to give 336 mg (41%) of methyl (E)-3-[2-n-butyl-1-{(2-chlorophenyl)methyl}-1H-imidazol-5-yl]-2-(2-thienyl)methyl-2-propenoate as an oil whose NMR was entirely consistent with the trans or F form of the olefin.

### (c) (E)-3-[2-n-butyl-1-{(2-chlorophenyl)methyl}-1H-imidazol-5-yl]-2-(2-thienyl)methyl-2-propenoic acid

A solution of methyl (E)-3-[2-n-butyl-1-{(2-chlorophenyl)methyl}-1H-imidazol-5-yl]-2-(2-thienyl)-methyl-2-propenoate (336 mg, 0.783 mmol) in ethanol (10 mL) was treated with 10% sodium hydroxide solution (4 mL), and the solution was stirred for 3 hours at 25°C. The pH was adjusted to 5 and a solid precipitated. The mixture was diluted with water, cooled and filtered to provide 309 mg of solid. A crystallization from ethyl acetate gave 195 mg (60%) of (E)-3-[2-n-butyl-1-{(2-chlorophenyl)methyl)-1H-imidazol-5-yl]-2-(2-thienyl)methyl-2-propenoic acid; mp 177-179°C.

### Method B

### (a) methyl 3-[2-n-butyl- 1-{(2-chlorophenyl)methyl}-1H-imidazol-5-yl]-3-hydroxy-2-(2-thienyl)methylpropanoate

To a solution of diisopropylamine (1.96 g, 0.0194 mol) in dry tetrahydrofuran (40 mL) held at -78°C under argon was added n-butyl lithium (7.3 mL, 0.0183 mol of 2.5 M in toluene) and the mixture was stirred for 10 minutes. Then, methyl 3-(2-thienyl)propanoate (2.83 g, 0.9166 mol) in tetrahydrofuran (2 mL) was added, and the mixture was stirred for 30 minutes at -78°C. A solution of 2-n-butyl-1(2-chlorophenyl)methyl-1H-imidazol-5-carboxaldehyde (3 g, 0.0111 mol) in tetrahydrofuran (4 mL) was added, and the resulting mixture was stirred at -78°C for 30 minutes. The reaction was partitioned between saturated ammonium chloride solution and ether, the organic extract was washed with brine, dried over anhydrous magnesium sulfate and concentrated to 6.67 g of crude product. This was flash chromatographed over 70 g of silica gel with 4:1 ethyl acetate/hexane to provide 4.03 g (81%) of methyl 3-[2-n-butyl-1-{(2-chlorophenyl)methyl}-1H-imidazol-5-yl]-3-hydroxy-2-(2-thienyl)methylpropanoate.

### (b) methyl 3-acetoxy-3-[2-n-butyl-1-{(2-chlorophenyl)methyl}-1H-imidazol-5-yl]-2-(2-thienyl)methylpropanoate

A solution of methyl 3-[2-n-butyl-1-{(2-chlorophenyl)methyl}-1H-imidazol-5-yl]-3-hydroxy-2-(2-thienyl)methylpropanoate (4.03 g, 9.02 mmol) in methylene chloride (100 mL) was treated with 4-dimethylaminopyridine (0.386 g, 3.16 mmol). Then acetic anhydride (8.5 mL, 9.02 mmol) was added dropwise to the stirred mixture. The mixture was stirred for 18 hours, water (35 mL) was added, the mixture was stirred for 1 hour and then diluted with ether and saturated sodium bicarbonate solution. The ether layer was washed with brine, dried with anhydrous magnesium sulfate and evaporated to give the title 3-acetoxy derivative as an oil (4.37 g, 99%).

### (c) methyl (E)-3-[2-n-butyl-1-{(2-chlorophenyl)methyl}-1H-imidazol-5-yl]-2-(2-thienyl)methyl-2-propenoate

A mixture of methyl 3-acetoxy-3-[2-n-butyl-1-{(2-chlorophenyl)methyl}-1H-imidazol-5-yl]-2-(2-thienyl)methylpropanoate (4.36 g, 8.92 mmol) in dry toluene (80 mL) was treated with 1,8-diazabicyclo[5,4,0]undec-7-ene (DBU) (3.2 mL, 21.4 mmol), and the resulting solution was heated at 80°C under argon for 3 hours. The solvent was evaporated, the residue triturated with ether and activated charcoal was added. After filtration, the filtrate was concentrated to 6.29 g of an oil that was chromatographed over silica gel with 65:35 hexane/ethyl acetate to give 2.89 g (76%) of methyl (E)-3-[2-n-butyl-1-{(2-chlorophenyl)methyl}-1H-imidazol-5-yl]-2-(2-thienyl)methyl-2-propenoate whose NMR and TLC (50% ethyl acetate in hexane on silica gel) were identical to the product prepared by Method A.

### (d) (E)-3-[2-n-butyl-1-{(2-chlorophenyl)methyl]}-1H-imidazol-5-yl]-2-(2-thienyl)methyl-2-propenoic acid

Basic hydrolysis of this ester (2.88 g, 6.71 mmol) according to Method A (iii) gave 2.59 g (93%) of (E)-3-[2-n-butyl-1-{(2-chlorophenyl)methyl}-1H-imidazol-5-yl]-2-(2-thienyl)methyl-2-propenoic acid; mp 175-177°C that was identical to the product from Method A.

### Example 2

### (E)-3-[2-n-Butyl-1-{(4-carboxyphenyl)methyl}-1H-imidazole-5-yl]-2-(2-thienyl)methyl-2-propenoic acid

By the procedure of Example 1 [(ii) method 2, (iii) and (iv) method B] using 4-carbomethoxybenzyl alcohol in place of 2-chlorobenzyl alcohol, the title compound was prepared; mp 250-253°C.

### Example 3

An oral dosage form for administering orally active Formula (I) compounds is produced by screening, mixing and filling into hard gelatin capsules the ingredients in proportions, for example, as shown below.

| Ingredients | Amounts |
|---|---|
| (E)-3-[2-n-butyl-1-{(4-carboxyphenyl)-methyl}-1H-imidazol-5-yl]-2-(2-thienyl)methyl-2-propenoic acid | 100 mg |
| magnesium stearate | 10mg |
| lactose | 100mg |

It is to be understood that the invention is not limited to the embodiments illustrated hereabove and the right to the illustrated embodiments and all modifications coming within the scope of the following claims is reserved.

## Claims (Claims for the following Contracting State(s): (AT, BE, CH, DE, DK, FR, GB, IT, LI, LU, NL, SE))

1. 3-[2-n-Butyl-1-{(4-carboxyphenyl)methyl}-1H-imidazol-5-yl]-2-(2-thienyl)methyl-2-propenoic acid, or a pharmaceutically acceptable salt thereof.

2. A compound according to claim 1 which is (E)-3-[2-n-butyl-1-{(4-carboxyphenyl)methyl]-1H-imidazol-5-yl]-2-(2-thienyl)methyl-2-propenoic acid, or a pharmaceutically acceptable salt thereof.

3. A compound according to claim 1 or 2 in which the pharmaceutically acceptable salt is the methane sulfonate salt.

4. A compound according to any one of claims 1 to 3 for use as a medicament.

5. A pharmaceutical composition which comprises a compound according to any one of claims 1 to 3 and a pharmaceutically acceptable carrier.

6. A process for preparing a compound according to any one of claims 1 to 3, which process comprises:
a) reacting a compound of the formula (II): 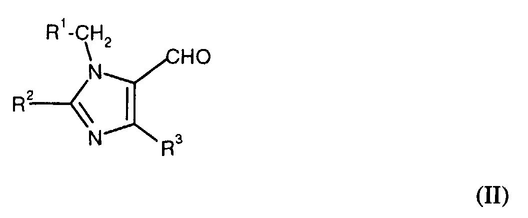 wherein R¹ is 4-(CO₂C₁₋₄alkyl)phenyl, R² is n-butyl, and R³ is hydrogen, with (C₁-C₄alkoxy)₂P(O)CH(R⁵)-COOC₁-C₆alkyl, wherein R⁵ is 2-thienyl-CH₂-, in the presence of a base; or
b) reacting a compound of the formula (III): 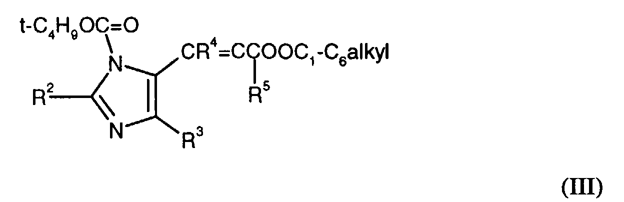 wherein R² is n-butyl, R³ is hydrogen, R⁴ is hydrogen, and R⁵ is 2-thienyl-CH₂-, with a compound of the formula (IV): 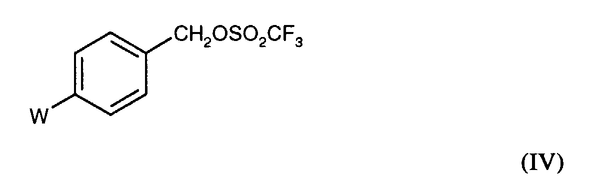 wherein W is CO₂C₁-C₄alkyl; or
c) reacting a compound of the formula (II) as hereinbefore defined with a compound of the formula (V): 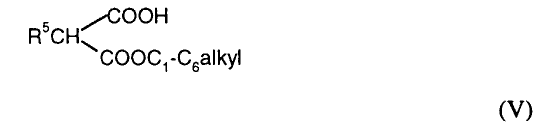 wherein R⁵ is 2-thienyl-CH₂-, in the presence of a base; or
d) reacting a compound of the formula (VI): 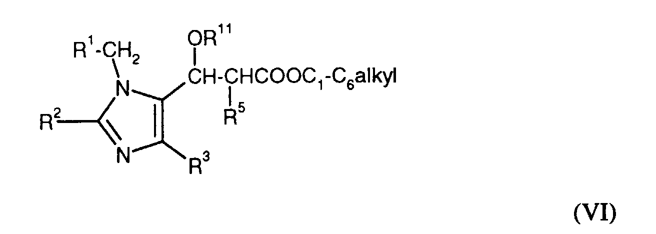 wherein R² is n-butyl, R³ is hydrogen, R⁵ is 2-thienyl-CH₂-, R¹ is 4-(CO₂C₁₋₄alkyl)phenyl, and R¹¹ is COCH₃ or SO₂CH₃, with a base; or
e) reacting a compound of the formula (II) as hereinbefore defined with an appropriate heterocyclic acetic acid in acetic anhydride in the presence of a base;
and thereafter:
(i) hydrolyzing the compounds in which the R¹ is 4-(CO₂C₁₋₄alkyl)phenyl; or
(ii) hydrolyzing the compounds in which R⁶ is -COOC₁-C₆alkyl;
and thereafter optionally forming a pharmaceutically acceptable salt.

7. The use of a compound as defined in any one of claims 1 to 3 in the manufacture of a medicament for treatment of diseases in which angiotensin II receptor antagonism is a factor.

8. The use of a compound as defined in any one of claims 1 to 3 in the manufacture of a medicament for treatment of hypertension.

9. The use of a compound as defined in any one of claims 1 to 3 in the manufacture of a medicament for treatment of congestive heart failure.

10. The use of a compound as defined in any one of claims 1 to 3 in the manufacture of a medicament for treatment of renal failure.

## Claims (Claims for the following Contracting State(s): ES, GR)

1. 3-[2-n-Butyl-1-{(4-carboxyphenyl)methyl}-1H-imidazol-5-yl]-2-(2-thienyl)methyl-2-propenoic acid, or a pharmaceutically acceptable salt thereof.

2. A compound according to claim 1 which is (E)-3-[2-n-butyl-1-{(4-carboxyphenyl)methyl}-1H-imidazol-5-yl]-2-(2-thienyl)methyl-2-propenoic acid, or a pharmaceutically acceptable salt thereof.

3. A compound according to claim 1 or 2 in which the pharmaceutically acceptable salt is the methane sulfonate salt.

4. A pharmaceutical composition which comprises a compound according to any one of claims 1 to 3 and a pharmaceutically acceptable carrier.

5. A process for preparing 3-[2-n-butyl-1-{(4-carboxyphenyl)methyl]-1H-imidazol-5-yl]-2-(2-thienyl)methyl-2-propenoic acid, or a pharmaceutically acceptable salt thereof, which process comprises:
a) reacting a compound of the formula (II): 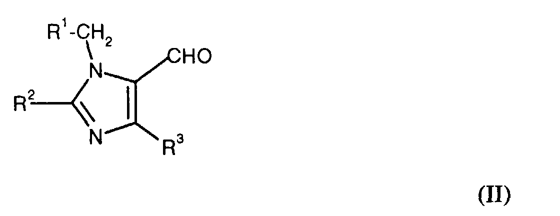 wherein R¹ is 4-(CO₂C₁₋₄alkyl)phenyl, R² is n-butyl, and R³ is hydrogen, with (C₁-C₄alkoxy)₂P(O)CH(R⁵)-COOC₁-C₆alkyl, wherein R⁵ is 2-thienyl-CH₂-, in the presence of a base; or
b) reacting a compound of the formula (III): 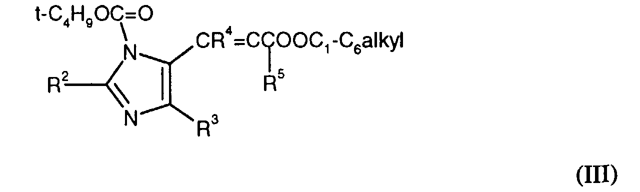 wherein R² is n-butyl, R³ is hydrogen, R⁴ is hydrogen, and R⁵ is 2-thienyl-CH₂-, with a compound of the formula (IV): 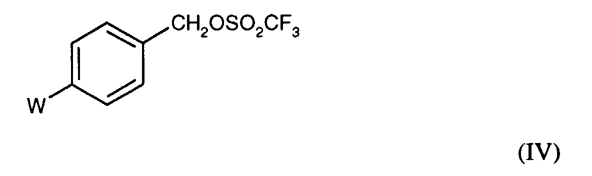 wherein W is CO₂C₁-C₄alkyl; or
c) reacting a compound of the formula (II) as hereinbefore defined with a compound of the formula (V): 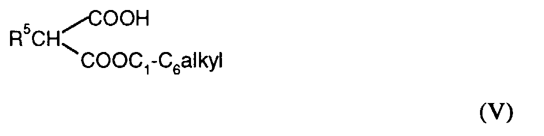 wherein R⁵ is 2-thienyl-CH₂-, in the presence of a base; or
d) reacting a compound of the formula (VI): 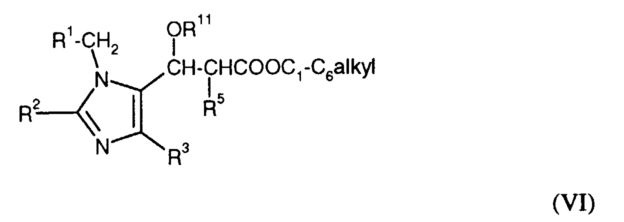 wherein R² is n-butyl, R³ is hydrogen, R⁵ is 2-thienyl-CH₂-, R¹ is 4-(CO₂C₁₋₄alkyl)phenyl, and R¹¹ is COCH₃ or SO₂CH₃, with a base; or
e) reacting a compound of the formula (II) as hereinbefore defined with an appropriate heterocyclic acetic acid in acetic anhydride in the presence of a base;
and thereafter:
(i) hydrolyzing the compounds in which the R¹ is 4-(CO₂C₁₋₄alkyl)phenyl; or
(ii) hydrolyzing the compounds in which R⁶ is -COOC₁-C₆alkyl;
and thereafter optionally forming a pharmaceutically acceptable salt.

6. A process according to claim 6 for preparing a compound which is (E)-3-[2-n-butyl-1-{(4-carboxyphenyl)methyl)-1H-imidazol-5-yl]-2-(2-thienyl)methyl-2-propenoic acid, or a pharmaceutically acceptable salt thereof.

7. A process according to claim 6 or 7 for preparing a compound in which the pharmaceutically acceptable salt is the methane sulfonate salt.

8. A process for preparing (E)-3-[2-n-butyl-1-{(4-carboxyphenyl)methyl}-1H-imidazol-5-yl]-2-(2-thienyl)methyl-2-propenoic acid, or a pharmaceutically acceptable salt thereof, which comprises reacting 3-acetoxy-3-[2-n-butyl-1-{(4-carbomethoxyphenyl)methyl}-1H-imidazol-5-yl]-2-(2-thienyl)methylpropanoate with 1,8-diazabicyclo[5.4.0]undec-7-ene, followed by ester hydrolysis, and thereafter optionally forming a pharmaceutically acceptable salt.

9. A process for preparing a pharmaceutical composition which comprises bringing into association 3-[2-n-butyl-1- {(4-carboxyphenyl)methyl}-1H-imidazol-5-yl]-2-(2-thienyl)methyl-2-propenoic acid, or a pharmaceutically acceptable salt thereof, and a pharmaceutically acceptable carrier.

10. The use of 3-[2-n-butyl-1-{(4-carboxyphenyl)methyl}-1H-imidazol-5-yl]-2-(2-thienyl)methyl-2-propenoic acid, or a pharmaceutically acceptable salt thereof, in the manufacture of a medicament for treatment of diseases in which angiotensin II receptor antagonism is a factor.

11. The use of 3-[2-n-butyl-1-{(4-carboxyphenyl)methyl}-1H-imidazol-5-yl]-2-(2-thienyl)methyl-2-propenoic acid, or a pharmaceutically acceptable salt thereof, in the manufacture of a medicament for treatment of hypertension.

12. The use of 3-[2-n-butyl-1-{(4-carboxyphenyl)methyl}-1H-imidazol-5-yl]-2-(2-thienyl)methyl-2-propenoic acid, or a pharmaceutically acceptable salt thereof, in the manufacture of a medicament for treatment of congestive heart failure.

13. The use of 3-[2-n-butyl-1-{(4-carboxyphenyl)methyl}-1H-imidazol-5-yl]-2-(2-thienyl)methyl-2-propenoic acid, or a pharmaceutically acceptable salt thereof, in the manufacture of a medicament for treatment of renal failure.

## Patentansprüche (Patentansprüche für folgende(n) Vertragsstaat(en): AT, BE, CM, DE, DK, FR, GB, IT, LI, LU, NL, SE)

1. 3-[2-n-Butyl-1-{(4-carboxyphenyl)methyl}-1H-imidazol-5-yl]-2-(2-thienyl)methyl-2-propensäure oder ein pharmazeutisch verträgliches Salz davon.

2. Verbindung nach Anspruch 1, nämlich (E)-3-[2-n-Butyl-1-{(4-carboxyphenyl)methyl}-1H-imidazol-5-yl]-2-(2-thienyl)methyl-2-propensäure oder ein pharmazeutisch verträgliches Salz davon.

3. Verbindung nach Anspruch 1 oder 2, wobei das pharmazeutisch verträgliche Salz das Methansulfonatsalz ist.

4. Verbindung nach einem der Ansprüche 1 bis 3 zur Verwendung als Medikament.

5. Arzneimittel, das eine Verbindung nach einem der Ansprüche 1 bis 3 und einen pharmazeutisch verträglichen Träger umfasst.

6. Verfahren zur Herstellung einer Verbindung nach einem der Ansprüche 1 bis 3, wobei das Verfahren umfasst:
a) Umsetzen einer Verbindung der Formel (II): 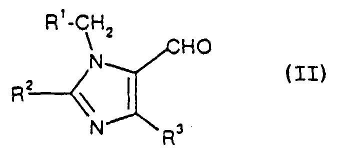 wobei R¹ ein 4-(CO₂C₁₋₄-Alkyl)phenylrest, R² eine n-Butylgruppe und R³ ein Wasserstoffatom ist, mit (C₁-C₄-Alkoxy)₂P(O)CH(R⁵)-COOC₁-C₆-Alkyl, wobei R⁵ ein 2-Thienyl-CH₂-Rest ist, in Gegenwart einer Base; oder
b) Umsetzen einer Verbindung der Formel (III): 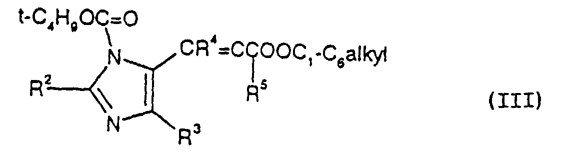 wobei R² eine n-Butylgruppe, R³ ein Wasserstoffatom, R⁴ ein Wasserstoffatom und R⁵ ein 2-Thienyl-CH₂-Rest ist, mit einer Verbindung der Formel (IV): 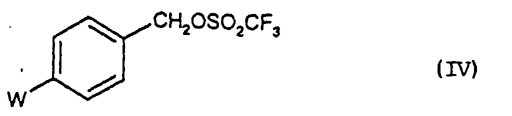 wobei W ein Rest CO₂C₁-C₄-Alkyl ist; oder
c) Umsetzen einer Verbindung der Formel (II), wie vorstehend definiert, mit einer Verbindung der Formel (V): 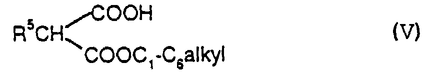 wobei R⁵ ein 2-Thienyl-CH₂-Rest ist, in Gegenwart einer Base; oder
d) Umsetzen einer Verbindung der Formel (VI): 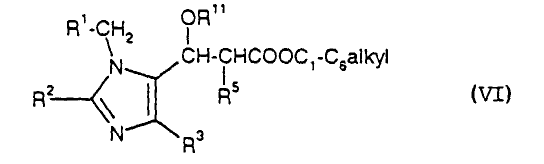 wobei R² eine n-Butylgruppe, R³ ein Wasserstoffatom, R⁵ ein 2-Thienyl-CH₂-Rest, R¹ ein 4-(CO₂C₁₋₄-Alkyl)phenylrest und R¹¹ eine Gruppe -COCH₃ oder -SO₂CH₃ ist, mit einer Base; oder
e) Umsetzen einer Verbindung der Formel (II), die wie vorstehend definiert ist, mit einer geeigneten heterocyclischen Essigsäure in Essigsäureanhydrid in Gegenwart einer Base;
und anschließendes
(i) Hydrolysieren der Verbindungen, bei denen R¹ ein 4-(CO₂C₁₋₄-Alkyl)phenylrest ist; oder
(ii) Hydrolysieren der Verbindungen, bei denen R⁶ ein -COOC₁-C₆-Alkyl-Rest ist;
und gegebenenfalls anschließendes Erzeugen eines pharmazeutisch verträglichen Salzes.

7. Verwendung einer Verbindung wie in einem der Ansprüche 1 bis 3 definiert zur Herstellung eines Medikaments zur Behandlung von Krankheiten, bei denen Angiotensin-II-Rezeptorantagonismus eine Rolle spielt.

8. Verwendung einer Verbindung wie in einem der Ansprüche 1 bis 3 definiert zur Herstellung eines Medikaments zur Behandlung von Bluthochdruck.

9. Verwendung einer Verbindung wie in einem der Ansprüche 1 bis 3 definiert zur Herstellung eines Medikaments zur Behandlung von dekompensierter Herzinsuffizienz.

10. Verwendung einer Verbindung wie in einem der Ansprüche 1 bis 3 definiert zur Herstellung eines Medikaments zur Behandlung von Nierenversagen.

## Patentansprüche (Patentansprüche für folgende(n) Vertragsstaat(en): ES, GR)

1. 3-[2-n-Butyl-1-{(4-carboxyphenyl)methyl}-1H-imidazol-5-yl]-2-(2-thienyl)methyl-2-propensäure oder ein pharmazeutisch verträgliches Salz davon.

2. Verbindung nach Anspruch 1, nämlich (E)-3-[2-n-Butyl-1-{(4-carboxyphenyl)methyl}-1H-imidazol-5-yl]-2-(2-thienyl)methyl-2-propensäure oder ein pharmazeutisch verträgliches Salz davon.

3. Verbindung nach Anspruch 1 oder 2, wobei das pharmazeutisch verträgliche Salz das Methansulfonatsalz ist.

4. Arzneimittel, das eine Verbindung nach einem der Ansprüche 1 bis 3 und einen pharmazeutisch verträglichen Träger umfasst.

5. Verfahren zur Herstellung von 3-[2-n-Butyl-1-{(4-carboxyphenyl)methyl}-1H-imidazol-5-yl]-2-(2-thienyl)methyl-2-propensäure oder eines pharmazeutisch verträglichen Salzes davon, wobei das Verfahren umfasst:
a) Umsetzen einer Verbindung der Formel (II): 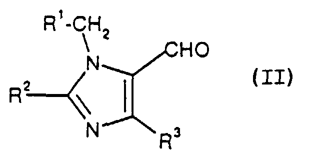 wobei R¹ ein 4-(CO₂C₁₋₄-Alkyl)phenylrest, R² eine n-Butylgruppe und R³ ein Wasserstoffatom ist, mit (C₁-C₄-Alkoxy)₂P(O)CH(R⁵)-COOC₁-C₆-Alkyl, wobei R⁵ ein 2-Thienyl-CH₂-Rest ist, in Gegenwart einer Base; oder
b) Umsetzen einer Verbindung der Formel (III): 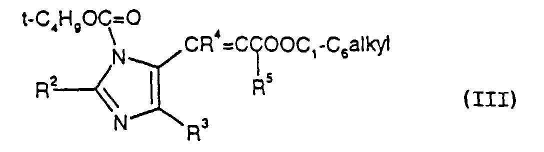 wobei R² eine n-Butylgruppe, R³ ein Wasserstoffatom, R⁴ ein Wasserstoffatom und R⁵ ein 2-Thienyl-CH₂-Rest ist, mit einer Verbindung der Formel (IV): 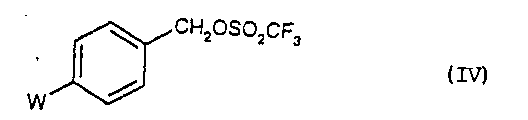 wobei W ein Rest CO₂C₁-C₄-Alkyl ist; oder
c) Umsetzen einer Verbindung der Formel (II), wie vorstehend definiert, mit einer Verbindung der Formel (V): 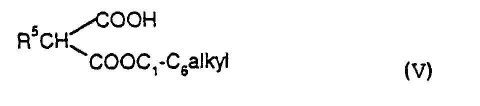 wobei R⁵ ein 2-Thienyl-CH₂-Rest ist, in Gegenwart einer Base; oder
d) Umsetzen einer Verbindung der Formel (VI): 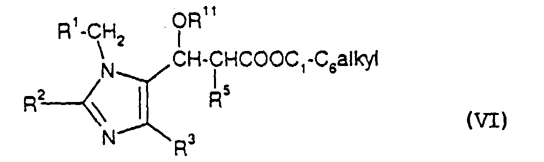 wobei R² eine n-Butylgruppe, R³ ein Wasserstoffatom, R⁵ ein 2-Thienyl-CH₂-Rest, R¹ ein 4-(CO₂C₁₋₄-Alkyl)phenylrest und R¹¹ eine Gruppe -COCH₃ oder -SO₂CH₃ ist, mit einer Base; oder
e) Umsetzen einer Verbindung der Formel (II), die wie vorstehend definiert ist, mit einer geeigneten heterocyclischen Essigsäure in Essigsäureanhydrid in Gegenwart einer Base;
und anschließendes
(i) Hydrolisieren der Verbindungen, bei denen R¹ ein 4-(CO₂C₁₋₄-Alkyl)phenylrest ist; oder
(ii) Hydrolisieren der Verbindungen, bei denen R⁶ ein -COOC₁-C₆-Alkyl-Rest ist; und gegebenenfalls anschließendes Bilden eines pharmazeutisch verträglichen Salzes.

6. Verfahren nach Anspruch 5 zur Herstellung einer Verbindung, nämlich (E)-3-[2-n-Butyl-1-{(4-carboxyphenyl)methyl}-1H-imidazol-5-yl]-2-(2-thienyl)methyl-2-propensäure, oder ein pharmazeutisch verträgliches Salz davon.

7. Verfahren nach Anspruch 5 oder 6 zur Herstellung einer Verbindung, bei der das pharmazeutisch verträgliche Salz das Methansulfonatsalz ist.

8. Verfahren zur Herstellung von (E)-3-[2-n-butyl-1-{(4-carboxyphenyl)methyl}-1H-imidazol-5-yl]-2-(2-thienyl)methyl-2-propensäure oder eines pharmazeutisch verträglichen Salzes davon, das das Umsetzen von 3-Acetoxy-3-[2-n-butyl-1-{(4-carbomethoxyphenyl)methyl}-1H-imidazol-5-yl]-2-(2-thienyl)methylpropionat mit 1,8-Diazobicylo[5.4.0]undec-7-en, nachfolgende Esterhydrolyse und gegebenenfalls anschließendes Bilden eines pharmazeutisch verträglichen Salzes, umfasst.

9. Verfahren zur Herstellung einer pharmazeutischen Zusammensetzung, welches das Zusammenbringen von 3-[2-n-Butyl-1-{(4-carboxyphenyl)methy}-1H-imidazol-5-yl]-2-(2-thienyl)methyl-2-propensäure oder eines pharmazeutisch verträglichen Salzes davon mit einem pharmazeutisch verträglichen Träger umfasst.

10. Verwendung von 3-[2-n-Butyl-1-{(4-carboxyphenyl)methyl}-1H-imidazol-5-yl]-2-(2-thienyl)methyl-2-propensäure oder eines pharmazeutisch verträglichen Salzes davon zur Herstellung eines Medikaments zur Behandlung von Krankheiten, bei denen Angiotensin-II-Rezeptorantagonismus eine Rolle spielt.

11. Verwendung von 3-[2-n-Butyl-1-{(4-carboxyphenyl)methyl}-1H-imidazol-5-yl]-2-(2-thienyl)methyl-2-propensäure oder eines pharmazeutisch verträglichen Salzes davon zur Herstellung eines Medikaments zur Behandlung von Bluthochdruck.

12. Verwendung von 3-[2-n-Butyl-1-{(4-carboxyphenyl)methyl}-1H-imidazol-5-yl]-2-(2-thienyl)methyl-2-propensäure oder eines pharmazeutisch verträglichen Salzes davon zur Herstellung eines Medikaments zur Behandlung von dekompensierter Herzinsuffizienz.

13. Verwendung von 3-[2-n-Butyl-1-{(4-carboxyphenyl)methyl}-1H-imidazol-5-yl]-2-(2-thienyl)methyl-2-propensäure oder eines pharmazeutisch verträglichen Salzes davon zur Herstellung eines Medikaments zur Behandlung von Nierenversagen.

## Revendications (Revendications pour l'(les) Etat(s) contractant(s) suivant(s): AT, BE, CH, DE, DK, FR, GB, IT, LI, LU, NL, SE)

1. Acide 3-[2-N-butyl-1-{(4-carboxyphényl)-méthyl}-1H-imidazole-5-yl]-2-(2-thiényl)méthyl-2-propénoïque, ou un de ses sels pharmaceutiquement acceptables.

2. Composé suivant la revendication 1, qui consiste en l'acide (E)-3-[2-n-butyl-1-{(4-carboxyphényl)-méthyl} -1H-imidazole-5-yl] -2- (2-thiényl)méthyl-2-propénoïque, ou un de ses sels pharmaceutiquement acceptables.

3. Composé suivant la revendication 1 ou 2, dans lequel le sel pharmaceutiquement acceptable est le méthane-sulfonate.

4. Composé suivant l'une quelconque des revendications 1 à 3, destiné à être utilisé comme médicament.

5. Composition pharmaceutique qui comprend un composé suivant l'une quelconque des revendications 1 à 3 et un support pharmaceutiquement acceptable.

6. Procédé pour la préparation d'un composé suivant l'une quelconque des revendications 1 à 3, procédé qui comprend :
a) la réaction d'un composé de formule (II) : 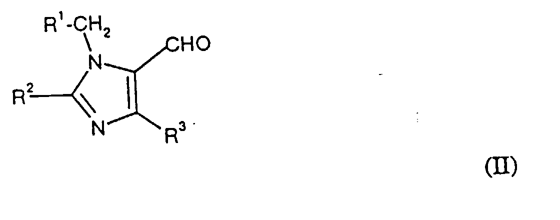 dans laquelle R¹ représente un groupe 4-(CO₂-(alkyle en C₁ à C₄) ) phényle, R² représente un groupe n-butyle et R³ représente l'hydrogène, avec un composé de formule (alkoxy en C₁ à C₄)₂P(O)CH(R⁵)-COO(alkyle en C₁ à C₆) dans lequel R⁵ représente un groupe 2-thiényl-CH₂-, en présence d'une base ; ou
b) la réaction d'un composé de formule (III) : 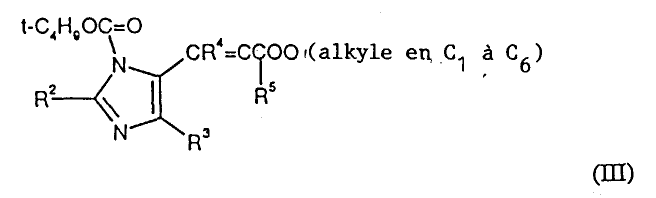 dans laquelle R² représente un groupe n-butyle, R³ représente l'hydrogène, R⁴ représente l'hydrogène et R⁵ représente un groupe 2-thiényl-CH₂-, avec un composé de formule (IV) : 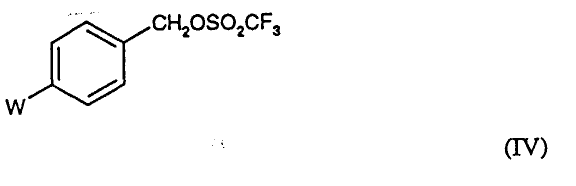 dans laquelle W représente un groupe CO₂(alkyle en C₁ à C₄) ; ou
c) la réaction d'un composé de formule (II) répondant à la définition précitée avec un composé de formule 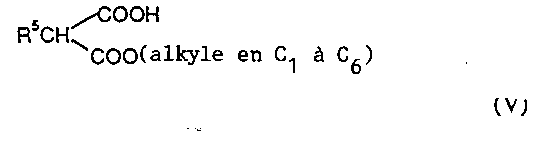 dans laquelle R⁵ représente un groupe 2-thiényl-CH₂-, en présence d'une base ; ou
d) la réaction d'un composé de formule (VI) : 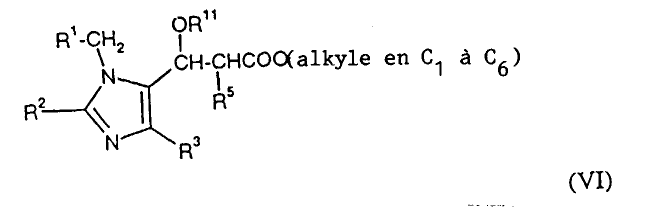 dans laquelle R² représente un groupe n-butyle, R³ représente l'hydrogène, R⁵ représente un groupe 2-thiényl-CH₂-, R¹ représente un groupe 4-(CO₂(alkyle en C₁ à C₄))-phényle et R¹¹ représente un groupe COCH₃ ou SO₂CH₃, avec une base ; ou
e) la réaction d'un composé de formule (II) répondant à la définition précitée avec un acide acétique hétérocyclique approprié dans de l'anhydride acétique en présence d'une base ;
et ensuite :
(i) l'hydrolyse des composés dans lesquels le groupe R¹ représente un groupe 4-(CO₂(alkyle en C₁ à C₄))-phényle ; ou
(ii) l'hydrolyse des composés dans lesquels R⁶ représente un groupe -COO(alkyle en C₁ à C₆) ;
puis, facultativement, la formation d'un sel pharmaceutiquement acceptable.

7. Utilisation d'un composé répondant à la définition suivant l'une quelconque des revendications 1 à 3 dans la production d'un médicament destiné au traitement de maladies dans lesquelles un facteur consiste en l'antagoniste des récepteurs d'angiotensine II.

8. Utilisation d'un composé répondant à la définition suivant l'une quelconque des revendications 1 à 3 dans la production d'un médicament destiné au traitement de l'hypertension.

9. Utilisation d'un composé répondant à la définition suivant l'une quelconque des revendications 1 à 3 dans la production d'un médicament destiné au traitement de l'insuffisance cardiaque congestive.

10. Utilisation d'un composé répondant à la définition suivant l'une quelconque des revendications 1 à 3 dans la production d'un médicament destiné au traitement de l'insuffisance rénale.

## Revendications (Revendications pour l'(les) Etat(s) contractant(s) suivant(s): ES, GR)

1. Acide 3-[2-N-butyl-1-{(4-carboxyphényl)-méthyl}-1H-imidazole-5-yl]-2-(2-thiényl)méthyl-2-propénoïque, ou un de ses sels pharmaceutiquement acceptables.

2. Composé suivant la revendication 1, qui consiste en l'acide (E)-3-[2-n-butyl-1-{(4-carboxyphényl)-méthyl}-1H-imidazole-5-yl]-2-(2-thiényl)méthyl-2-propénoïque, ou un de ses sels pharmaceutiquement acceptables.

3. Composé suivant la revendication 1 ou 2, dans lequel le sel pharmaceutiquement acceptable est le méthane-sulfonate.

4. Composition pharmaceutique qui comprend un composé suivant l'une quelconque des revendications 1 à 3 et un support pharmaceutiquement acceptable.

5. Procédé pour la préparation de l'acide 3-[2-n-butyl-1-{(4-carboxyphényl)méthyl}-1H-imidazole-5-yl]-2-(2-thiényl)méthyl-2-propénoïque, ou d'un de ses sels pharmaceutiquement acceptables, procédé qui comprend :
a) la réaction d'un composé de formule (II) : 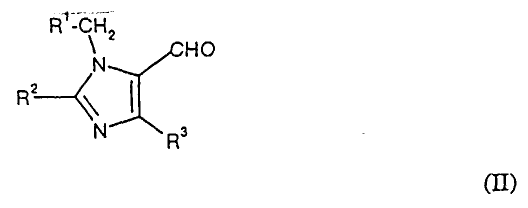 dans laquelle R¹ représente un groupe 4-(CO₂-(alkyle en C₁ à C₄) )phényle, R² représente un groupe n-butyle et R³ représente l'hydrogène, avec un composé de formule (alkoxy en C₁ à C₄)₂P(O)CH(R⁵)-COO(alkyle en C₁ à C₆) dans lequel R⁵ représente un groupe 2-thiényl-CH₂-, en présence d'une base ; ou
b) la réaction d'un composé de formule (III) : 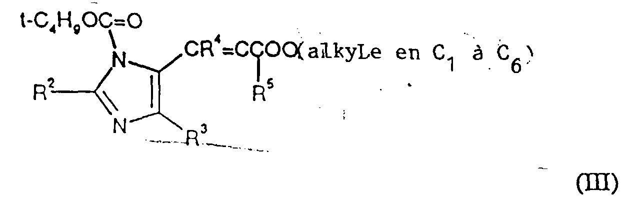 dans laquelle R² représente un groupe n-butyle, R³ représente l'hydrogène, R⁴ représente l'hydrogène et R⁵ représente un groupe 2-thiényl-CH₂-, avec un composé de formule (IV) : 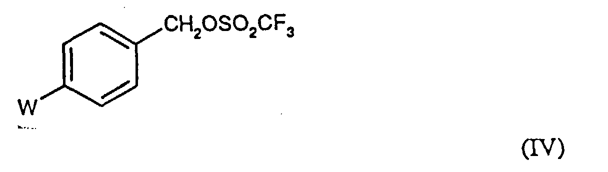 dans laquelle W représente un groupe CO₂ (alkyle en C₁ à C₄) ; ou
c) la réaction d'un composé de formule (II) répondant à la définition précitée avec un composé de formule 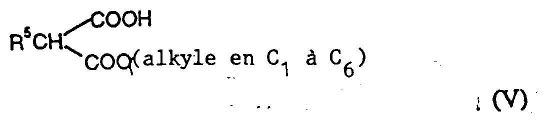 dans laquelle R⁵ représente un groupe 2-thiényl-CH₂-, en présence d'une base ; ou
d) la réaction d'un composé de formule (VI) : 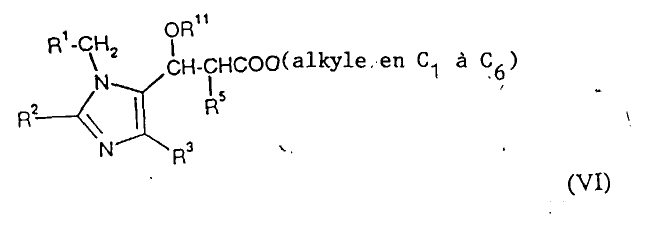 dans laquelle R² représente un groupe n-butyle, R³ représente l'hydrogène, R⁵ représente un groupe 2-thiényl-CH₂-, R¹ représente un groupe 4-(CO₂(alkyle en C₁ à C₄))-phényle et R¹¹ représente un groupe COCH₃ ou SO₂CH₃, avec une base ; ou
e) la réaction d'un composé de formule (II) répondant à la définition précitée avec un acide acétique hétérocyclique approprié dans l'anhydride acétique en présence d'une base ;
et ensuite :
(i) l'hydrolyse des composés dans lesquels le groupe R¹ est un groupe 4-(CO₂(alkyle en C₁ à C₄))phényle ; ou
(ii) l'hydrolyse des composés dans lesquels R⁶ représente un groupe -COO(alkyle en C₁ à C₆) ;
puis, facultativement, la formation d'un sel pharmaceutiquement acceptable.

6. Procédé suivant la revendication 5, pour la préparation d'un composé qui consiste en l'acide (E)-3-[2-n-butyl-1-{(4-carboxyphényl)méthyl}-1H-imidazole-5-yl]-2-(2-thiényl)méthyl-2-propénoïque, ou d'un de ses sels pharmaceutiquement acceptables.

7. Procédé suivant la revendication 5 ou 6, pour la préparation d'un composé dans lequel le sel pharmaceutiquement acceptable est le méthanesulfonate.

8. Procédé pour la préparation de l'acide (E)-3-[2-n-butyl-1-{(4-carboxyphényl)méthyl}-1H-imidazole-5-yl]-2-(2-thiényl)méthyl-2-propénoïque ou d'un de ses sels pharmaceutiquement acceptables, qui comprend la réaction du 3-acétoxy-3-[2-n-butyl-1-{(4-carbométhoxyphényl)méthyl}-1H-imidazole-5-yl}-2-(2-thiényl)méthylpropanoate avec le 1,8-diazabicyclo[5.4.0]undéc-7-ène, puis l'hydrolyse de l'ester et ensuite, facultativement, la formation d'un sel pharmaceutiquement acceptable.

9. Procédé pour la préparation d'une composition pharmaceutique, qui comprend la mise en association de l'acide 3-[2-n-butyl-1-{(4-carboxyphényl)méthyl}-1H-imidazole-5-yl]-2-(2-thiényl)méthyl-2-propénoïque ou d'un de ses sels pharmaceutiquement acceptables et d'un support pharmaceutiquement acceptable.

10. Utilisation de l'acide 3-[2-n-butyl-1-{(4-carboxyphényl)méthyl}-1H-imidazole-5-yl]-2-(2-thiényl)méthyl-2-propénoïque ou d'un de ses sels pharmaceutiquement acceptables dans la production d'un médicament destiné au traitement de maladies dans lesquelles un facteur consiste en l'antagoniste des récepteurs d'angiotensine II.

11. Utilisation de l'acide 3-[2-n-butyl-1-{(4-carboxyphényl)méthyl}-1H-imidazole-5-yl]-2-(2-thiényl)méthyl-2-propénoïque ou d'un de ses sels pharmaceutiquement acceptables dans la production d'un médicament destiné au traitement de l'hypertension.

12. Utilisation de l'acide 3-[2-n-butyl-1-{(4-carboxyphényl)méthyl}-1H-imidazole-5-yl]-2-(2-thiényl)méthyl-2-propénoïque ou d'un de ses sels pharmaceutiquement acceptables dans la production d'un médicament destiné au traitement de l'insuffisance cardiaque congestive.

13. Utilisation de l'acide 3-[2-n-butyl-1-{(4-carboxyphényl)méthyl}-1H-imidazole-5-yl]-2-(2-thiényl)méthyl-2-propénoïque ou d'un de ses sels pharmaceutiquement acceptables dans la production d'un médicament destiné au traitement de l'insuffisance rénale.
